**Europäisches Patentamt**

**European Patent Office**

**Office européen des brevets**

(19)

(11) Publication number: **0 122 361**
**B1**

# (12) EUROPEAN PATENT SPECIFICATION

(45) Date of publication of patent specification: **25.03.87**

(21) Application number: **84100137.3**

(22) Date of filing: **21.07.80**

(60) Publication number of the earlier application in accordance with Art. 76 EPC: **0 032 514**

(51) Int. Cl.⁴: **G 01 S 15/89**, G 10 K 11/30, A 61 B 8/14

(54) Ultrasonic wave tomographic imaging system.

(30) Priority: **25.07.79 JP 94532/79**

(43) Date of publication of application:
**24.10.84 Bulletin 84/43**

(45) Publication of the grant of the patent:
**25.03.87 Bulletin 87/13**

(84) Designated Contracting States:
**DE FR GB SE**

(56) References cited:
**JP-A-52 131 679**
**JP-A-53 032 987**
**US-A-3 913 061**
**US-A-3 937 066**
**US-A-4 131 021**

(73) Proprietor: **FUJITSU LIMITED**
**1015, Kamikodanaka Nakahara-ku**
**Kawasaki-shi Kanagawa 211 (JP)**

(72) Inventor: **Miyazaki, Junji**
**1462-12, Kobuchi**
**Sagamihara-shi Kanagawa 229 (JP)**
Inventor: **Miwa, Hirohide**
**6-7-10, Miyazaki Takatsu-ku**
**Kawasaki-shi Kanagawa, 213 (JP)**
Inventor: **Shimura, Takaki**
**29-44, Tsurukawa 4-chome**
**Machida-shi Tokyo, 194-01 (JP)**

(74) Representative: **Muir, Ian R. et al**
**HASELTINE LAKE & CO. Hazlitt House 28**
**Southampton Buildings Chancery Lane**
**London WC2A 1AT (GB)**

Courier Press, Leamington Spa, England.

# 0 122 361

**Description**

This invention relates to an ultrasonic wave tomographic imaging system which radiates an ultrasonic wave into an object and reproduces the internal conditions of said object as an image from the reflected wave.

An ultrasonic wave tomographic imaging system radiates an ultrasonic wave into an object of which the internal conditions are to be studied, receives the reflected wave returning from the inside of the object and produces therefrom an internal accoustic image of the object.

As a system for observing internal conditions in an object, X-ray diagnostic systems are widely used. An ultrasonic wave tomographic imaging system, as compared with such an X-ray diagnostic system, is, especially in the case of a human body as the object, non-destructive of internal organs and is less dangerous. Moreover, the ultrasonic system has the merit that it is suited to the diagnosis of soft organs of the human body.

As an ultrasonic wave tomographic imaging system, camera systems utilizing ultrasonic wave lenses have already been proposed. One such is described in U.S. Patent 4,131,023 which discloses a system in which the transmitted and received signals are passed through a rotating prism to effect scanning of a target. Another such system is disclosed in U.S. Patent 4,131,021 in this arrangement to partially compensate for spatial deviation between the locations of transmitted and received signals caused by rotation of the prisms transmission is effected from two spaced transducer elements, one in one half cycle and the other in the other half cycle with a common receiving spaced apart transducer. This arrangement can only compensate when used for a limited range of prism rotation or if the receiver array is very wide and hence open to receipt of noise signals.

The present invention seeks to provide an improved tomographic imaging system of a kind similar to that of U.S. Patent 4,131,021.

According to the present invention there is provided an ultrasonic wave tomographic imaging system incorporating ultrasonic wave transducer means for both radiating ultrasonic wave signals into an object and for receiving reflected ultrasonic wave signals from the object, an ultrasonic lens arrangement for focussing the radiated signals onto a desired tomographic plane of the object and for focussing signals reflected from the plane onto the transducer means, rotating prism means which, on rotation, cause said tomographic plane to be scanned by the focussed radiated signals, and compensating means to compensate for the deviation in the focussed beam received at the transducer means resulting from the rotation of the prisms means in the time between the passage of the radiated and the reflected wave signals therethrough, characterised in that the compensating means change the effective receiving area on the transducer means in accordance with the rotation of the prisms.

The compensating means may be formed by a slotted gating plate between the lens and the transducer arrangement; the plate being arranged to be shifted appropriately with respect to the rotation of the prism means so as to permit the desired received signals to pass through the slot to the transducer means.

Alternatively, the transducer means is formed as a raster array and the compensating means ensures that only the outputs from the transducers on which the desired signals are focussed, are utilised.

Preferably timing gate means are used to isolate the desired signals from undesired noise signals. That is the received signals are gated with the gate opening at the time required for signals to travel from the transducer to the focussed plane and back and with the gate shutting after a time corresponding to the transmitted pulse duration.

In systems according to this invention, the image plane is regionally radiated by the ultrasonic wave. With the availabilitiy of such regional radiation systems, the entire part may at first be diagnosed and thereafter the ultrasonic wave is regionally radiated only to the region to be examined carefully. The space noise of other non-radiated regions can, therefore, be avoided so as to make clearer the ultrasonic wave image obtained. For example, in the case of diagnosis of nephrolith, the ultrasonic wave is radiated at first to the entire part in order to obtain the total image and then radiated only to the region to be examined of the calculus. Thereby only a minute calculus becomes detectable by limiting the space noises reflected from the other regions such as ribs and diaphragm.

Moreover, this regional radiation is useful for combining an image of the entire part with less noise by selecting several regional images of the area to be examined. For this purpose, the tomographic plane is often scanned spot by spot with ultrasonic waves or scanned with a flat ultrasonic wave beam.

For a better understanding of the present invention reference will now be made, by way of example, to the accompanying drawings in which:

Figure 1 diagrammatically illustrates an ultrasonic wave tomographic imaging system of the kind with which the invention is concerned.

Figure 2 shows diagrammatically features of a system in accordance with the invention;

Figure 3 shows an alternative arrangement to features shown in Figure 2, and

Figure 4 shows diagrammatically an ultrasonic wave tomographic imaging system according to the invention.

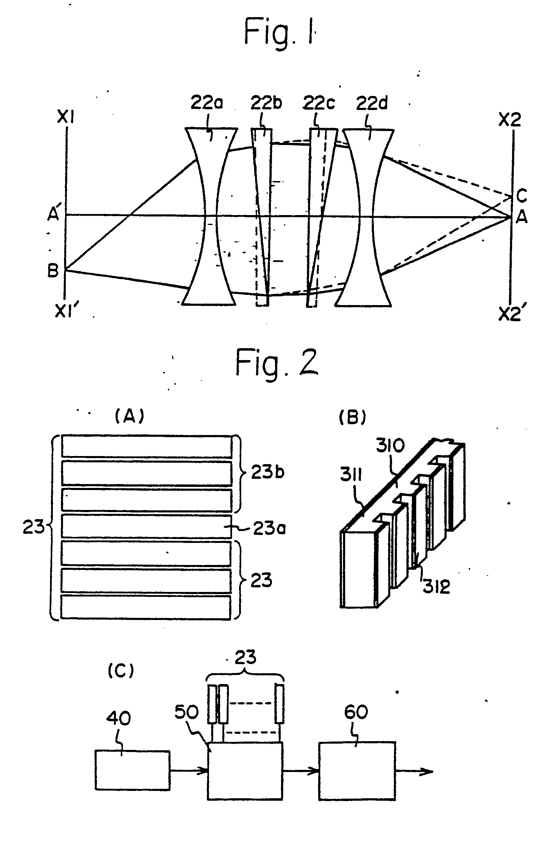

Figure 1 illustrates a focussed ultrasonic wave tomographic imaging system using scanning by means of rotating prisms. In this Figure, 22a and 22d are acoustic lenses; 22b and 22c are comb-shaped prisms;

2

$X_1$, $X_1'$ is an internal cross-section of an object to be scanned and imaged and $X_2$, $X_2'$ is a picture-forming surface, i.e. the reflected wave receiving and detecting surface.

In this system, a piezoelectric converter is provided vertically to the paper surface at the position A and the pair of comb-shaped prisms 22b and 22c are rotated so that an acoustic image moves reciprocally in the vertical direction $X_2$, $X_2'$ about the point A. When ultrasonic pulses are sequentially generated from the point A, these pulses are focussed and deflected by the lenses 22a and 22d and the prisms 22b and 22c, and sequentially radiated to each point to be scanned of the cross-section $X_1$, $X_1'$. If the pulse interval between pulses generated by a piezoelectric element is set so that all piezoelectric elements respectively send and receive sequentially once in the length of time of a pulse interval, and adjustment is made so that each piezoelectric element sends and receives a number of pulses equal to the number of scanning lines of the display unit whilst a prism rotates through 180 degrees, then one frame of a picture of the scanned area can be obtained during such a 180° prism rotation.

In this embodiment, the ultrasonic wave is regionally radiated to the cross-section $X_1$, $X_1'$ of an object, and therefore a strong reflected wave, resulting in a clear image can be obtained from this cross-section region with less noise in the image caused by reflections from other regions of the object. In addition, since a piezoelectric converter (transducer) is used for both transmission and reception of ultrasonic waves, the structure of the system is simplified.

However, if the ultrasonic wave transmitted from the point A is focussed on the point B, the reflected wave will not return to the point A but to another point slightly shifted from the point A, the point C, for example, since a prism will already have made a small rotation from the transmitted wave position prior to the reflected wave from the point B passing through the prism. Therefore, it is necessary to use, in the illustrated arrangement, piezoelectric converter having a sufficient depth to still receive the return wave despite the shift in its focussing position. Assuming that the transmission rate of the ultrasonic wave and the prism rotating speed are respectively considered as constant, the extent of shift of the focussing position, as explained above, depends on the distance from the prism to the cross-section of the object and the distance from the prism to the picture forming surface. The afore-mentioned distances have the mutual relationship that as one becomes short, the other becomes long. Therefore, for a particular angular rotation, the shift is almost constant irrespective of whether the region to be examined is deep or shallow in the object.

This shift can be ignored if the piezoelectric transducing means is sufficiently wide or deep, but a wide transducer may also pick up noise signals. Therefore, according to the invention, to overcome this problem, compensating means are provided to compensate for this shift or deviation of the focussing position on the piezoelectric transducer of the reflected wave. Three means of compensation are hereinafter described.

As compensating means selective reception on multiple elements of the piezoelectric transducer means may be employed and will be described first. In Figure 2(A), a plurality of individual transducer units 23a to 23b (7 units in this case) are arranged in parallel and used as the piezoelectric transducer means 23 and the transducer unit that is located in the focussing area is selected electrically in accordance with the shift of the focussing position. As an alternative, the transducer means could be formed as shown in Figure 2(B), where the common electrode 311 is provided for one side of a long and slender piezoelectric plate 310, while individual electrodes 312 are formed on projecting areas of the other side of the plate. As the electrical selection means to be used with such transducer means, there is provided a selection circuit 50 which sequentially selects, as necessary, the transducer element or unit of the transducer 23 as indicated in Figure 2(C), so that a transducer unit is selected for reception in accordance with the rotating angle information sent from a prism rotating angle detector 40. The received signal from the selected transducer unit is sent to a signal processing circuit 60 and converted to a picture signal therein for display on a CRT. Since for constant speed of rotation the shift of the focussing point, due to the change of prism angle changes according to a sine function with respect to time, selection of the transducer units by the selection circuit 50 should be made at timing points which change according to the sine function. In this method of compensation, it is possible to transmit the ultrasonic wave from the transducer unit 23a and receive it on the appropriate transducer unit of units 23 and 23b. When selective reception is effected by a narrow single transducer element according to this proposal, incoming noise can be suppressed outstandingly as compared with reception using a single piezoelectric transducer having a width or depth equivalent to that of the combination of the seven units.

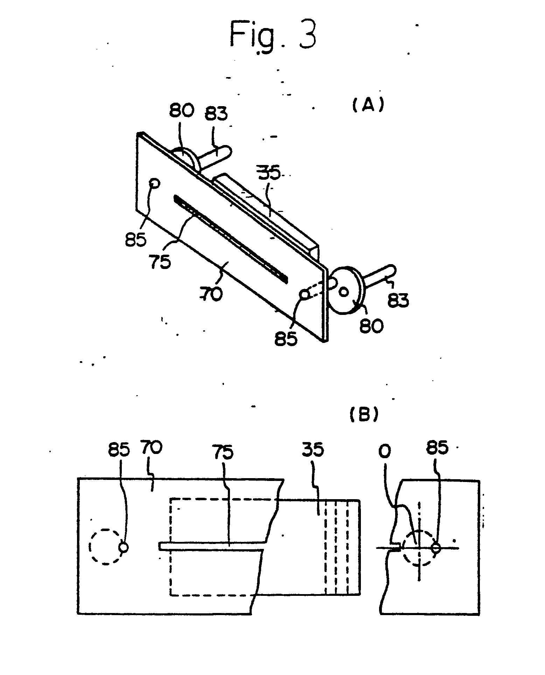

A second compensation arrangement is shown in Figure 3, where a slit mechanism compensating means is employed. As the piezoelectric transducer means, a wide or deep transducer unit 35 is used. A compensating mask plate 70 having a slot or slit 75 is provided in front of the transducer unit 35, and this mask plate is moved in conjunction with rotating disks 80 by means of pins 85. The rotating disks 80 are rotated in sychronization with the rotation of the prism means via electric motor rotating axes 83. Therefore, the slit 75 follows a reciprocal motion vertically in accordance with a sine function. Each element of the conversion unit may transmit or receive ultrasonic waves but the reception is effected selectively since only the area instantaneously exposed by the slit will receive ultrasonic waves.

A third possible method of compensation is to control the rotating speed of the prism to cause the focussing point to be shifted reciprocally at a constant speed (instead of as a sine function) and to select the

transducer unit according to this timing by using the piezoelectric transducer and selection circuit means of the first-described method and compensation.

A method for controlling the rotating speed of the prism means will now be explained.

When a prism rotates at a constant speed the shift in the focussing point at the picture forming surface $X_2$, $X_2'$ can be expressed by the following equation.

$$y = A_o \cdot \sin W_o t \qquad (1)$$

Where, t is time, $W_o$ is the uniform angular speed of the prism, and $A_o$ is amplitude. When the rotating speed of the prism means is considered as the function W(t) with $\int W(t)dt = \theta(t)$, the shift of the focussing point is considered to change linearly,

if it expressed as

$$y = B_o \cdot \sin\theta(t) = Kt \qquad (2)$$

Where, K is a proportional constant, $B_o$ is an amplitude, and $\theta(t)$ should be within the following range.

$$0 \leq \theta(t) \leq \pi/2$$

From equation (2),

$$\theta(t) = \sin^{-1} Kt/B_o \qquad (3)$$

When equation (3) is differentiated, an angular speed of prism is obtained as expressed below.

$$W(t) = d\theta(t)/dt = 1/\sqrt{(B_o/K)^2 - t^2} \qquad (4)$$

Therefore, when $t = B_o/K$, which satisfies equation (3) when $\theta(t)$ equals $\pi/2$, the value of W(t) in equation (4) becomes infinite. Since it is impossible to produce infinite angular speed, it is obviously impossible to linearly control the shift of the focussing point until the angle of rotation becomes $\pi/2$. Therefore, $B_o$ is set larger than $A_o$, and y is considered to change at a constant speed until the value of equation (2) becomes equal to $A_o$, and thereafter a different control of the angular speed is performed.

Various kinds of other control methods are considered, but rotation should be continued keeping the angular speed occurring at the time when y becomes $A_o$.

At this time, the angular speed is given by the following equation.

$$[W(t)]_t = A_{o/K} = [d\theta(t)/dt]_t = A_{o/K} = (K/A_o)/\sqrt{n^2 - 1}$$

Where,

$$B_o = n \cdot A_o \quad (n > 1) \qquad (6)$$

The prism means will have rotated for the angle $\theta(A_{O/K})$ when y reaches the value $A_o$ at the time $A_o/K$ and from the equations (3) and (6),

$$\theta(A_o/K) = \sin^{-1}(1/n) \qquad (7)$$

Therefore, the angle for which rotation should be made at a constant speed $[W(t)]_t = A_{o/K}$ is as follows.

$$\pi/2 - \theta(A/K) = \pi/2 - \sin^{-1}(1/n) \qquad (8)$$

If the time for the angle of rotation $\theta(t)$ to change from 0 to $\pi/2$ is equal to the time $\pi/2W_o$ for rotation at the constant angular speed, $W_o$ then, since the equation (8) is equal to the equation (5)×time $(\pi/2W_o - A_o/K)$, the following relation can be obtained.

$$(\pi/2W_o - A_oK) \ (K/A_o)/\sqrt{n^2 - 1} = \pi/2 - \sin^{-1}(1/n)$$

$$\therefore K = 2W_o/\pi \cdot A_o[\sqrt{n^2 - 1}\{\pi/2 - \sin^{-1}(1/n)\} + 1] \qquad (9)$$

Here, when n=2, from the equation (9),

$$K = 2W_o/\pi \cdot A_o \cdot (\pi/3 + 1) \qquad (10)$$

From the equation (6), $B_o = 2A_o$

When the following equation obtained from (10) is substituted to the equation (4),

$$B_o/K = 2A_o/K = \pi/W_o(\pi/\sqrt{3} + 1)$$

then

$$W(t) = \{(\pi/W_o(\pi/\sqrt{3} + 1))^2 - t^2\}^{-1/2}, \qquad (11)$$

where,

$$0 \leq t \leq A_o/K.$$

4

In other areas, rotation speed should be constant until the time $B_0/K$. Analysis can also be done in the same way even after the time $B_0/K$. Namely, all that is required is rotation control as expressed by the equation (11).

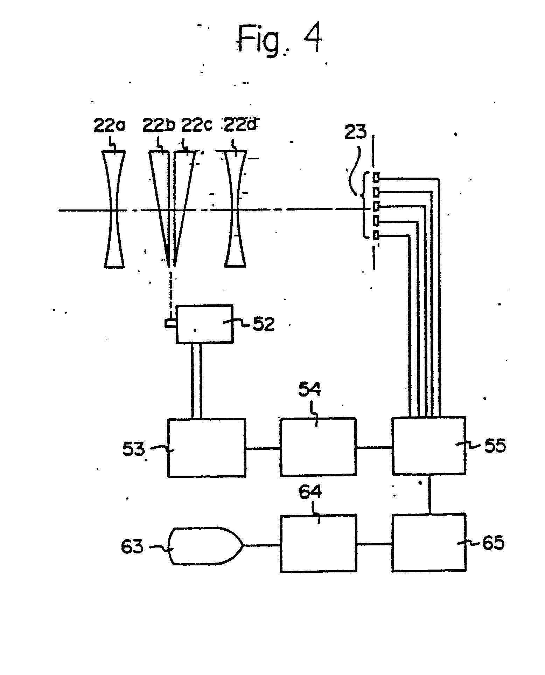

Figure 4 shows one embodiment of a circuit arrangement for putting into effect the above third-described method of compensation. The above-mentioned rotating angle $\theta(t)$ or angular speed $W(t)$ is programmed with respect to time in the rotation control circuit 54, and the pulse motor drive circuit 53 controlled in accordance with this programmed signal drives a pulse motor 52 causing the prisms 22b and 22c to rotate at the programmed rotating speed. At the same time, programmed prism rotation angle data is sent to a selective drive or gate circuit 55 for the transducer units 23, from the rotation control circuit 54, whereby transducer units are selected for transmission and reception of the ultrasonic waves. The received signal is converted to a picture signal in signal processing circuit 65 and displayed on CRT 63 through a display control circuit 64.

Moreover, this invention is capable of obtaining an acoustic image from a different tomographic plane by making use of the regional radiation method. Namely, the ultrasonic wave is regionally radiated, i.e. focussed on a tomographic plane but, if the focal depth of the lens is deep, acoustic energy which is almost the same as that radiated to the focussed plane region is radiated also to the adjacent regions on the generator side or on the opposite side of that plane in the vicinity of said focussed region. There is a time difference that occurs, of course, in the receiving timings of the waves reflected from these adjacent regions as compared with the waves from the focussed plane region. This invention has succeeded in freely changing the selected receiving wave timings using such time differences, so as to display clearly images of the tomographic planes near to that of the focussed region.

To isolate the image to the image of the desired plane, the ultrasonic wave receiving means is so structured that only the ultrasonic wave image of the desired regionally radiated plane of the tomographic plane can be received. For this purpose, for two-dimensional and flat ultrasonic wave receiving means, a function or electrical space gate means such as will synchronize with the sequential radiation is provided so that only the area on the image-forming surface corresponding to the desired regionally radiated area either receives ultrasonic picture waves or outputs an electrical signal of such ultrasonic image therefrom. In the same way, a mechanical space gate means which synchronizes with the sequential radiation is provided for the single dimensional ultrasonic wave receiving means.

The ultrasonic wave receiving means includes timing gate means to gate the received signals to eliminate space noise occurring outside the gated time, the timing gate means being so designed as to operate in synchronization with the correct time required for the ultrasonic wave to reach the ultrasonic wave receiving means from the generator after reflection.

**Claims**

1. An ultrasonic wave tomographic imaging system incorporating ultrasonic wave transducer means (23) for both radiating ultrasonic wave signals into an object and for receiving reflected ultrasonic wave signals from the object, an ultrasonic lens arrangement (22a, 22d) for focussing the radiated signals onto a desired tomographic plane $(X_1, X_1')$ of the object and for focussing signals reflected from the plane onto the transducer means (23), rotating prism means (22b, 22c) which, on rotation, cause said tomographic plane $(X_1, X_1')$ to be scanned by the focussed radiated signals, and compensating means (23b; 70, 75) to compensate for the deviation in the focussed beam received at the transducer means (23) resulting from the rotation of the prism means (22b, 22c) in the time between the passage of the radiated and the reflected wave signals therethrough, characterised in that the compensating means change the effective receiving area on the transducer means in accordance with the rotation of the prisms.

2. An ultrasonic wave tomographic imaging system according to Claim 1 wherein said compensating means is formed by a slotted gating plate (70) between the transducer means (23) and said lens arrangement (22a, 22d), and said plate (70) is arranged to be shifted appropriately with respect to the rotation of the prism means (22b, 22c) so as to permit the desired received signals to pass through the slot (75) to the transducer means (23).

3. An ultrasonic wave tomographic imaging system according to Claim 1 wherein the transducer means (23) is formed as a raster array of transducer elements (23, 23a, 23b) and wherein the compensating means ensures that only the outputs from the transducer elements in the effective area on which the desired signals are focussed are passed.

4. An ultrasonic wave tomographic imaging system according to any preceding claim including timing gate means to isolate desired signals from noise signals.

**Patentansprüche**

1. Ultraschall-Tomographie-Abbildungssystem, mit einer Ultraschallwellen-Wandlereinrichtung (23), sowohl zum Ausstrahlen von Ultraschallwellensignalen in ein Objekt als auch zum Empfang von reflektierten Ultraschallwellensignalen von dem Objekt, einer Ultraschall-Linsenanordnung (22a, 22d), zum Fokussieren der ausgestrahlten Signale auf eine gewünschte tomographische Ebene $(X_1, X_1')$ des Objektes und zum Fokussieren von Signalen, die von der Ebene auf die Wandlereinrichtung (23) reflektiert werden, rotierenden Prismeneinrichtungen (22b, 22c), die, bei Rotation, bewirken, daß die genannte tomographische Ebene ($X_1$, $X_1'$) von den fokussierten ausgestrahlten Signalen überstrichen wird, und Kompensationseinrichtung (23b; 70, 75), um die Abweichung des fokussierten Strahls, der bei der Wandlereinrichtung (23) empfangen wird, zu kompensieren, die von der Rotation der Prismeneinrichtungen (22b, 22c) in der Zeit zwischen dem Durchgang der ausgestrahlten und der reflektierten Wellensignale durch sie resultiert, dadurch gekennzeichnet, daß die Kompensationseinrichtung den effektiven Empfangsbereich der Wandlereinrichtung in Übereinstimmung mit der Rotation des Prismas ändert.

2. Ultraschall-Tomographie-Abbildungssystem nach Anspruch 1, bei dem die genannte Kompensationseinrichtung durch eine geschlitzte Austastplatte (70) zwischen der Wandlereinrichtung (23) und der genannten Linsenanordnung (22a, 22d) gebildet ist, und die genannte Platte (70) angeordnet ist, um in geeigneter Weise in bezug auf die Rotation der Prismeneinrichtung (22b, 22c) so verschoben zu werden, um zu erlauben, daß die gewünschten empfangenen Signale durch den Schlitz (75) zu der Wandlereinrichtung (23) hindurchgelangen.

3. Ultraschall-Tomographie-Abbildungssystem nach Anspruch 1, bei dem die Wandlereinrichtung (23) als eine Rasteranordnung von Wandlerelementen (23, 23a, 23b) gebildet ist und bei dem die Kompensationseinrichtung gewährleistet, daß lediglich die Ausgänge von den Wandlerelementen in dem effektiven Bereich, auf den die gewünschten Signale fokussiert sind, hindurchgelassen werden.

4. Ultraschall-Tomographie-Abbildungssystem nach einem der vorhergehenden Ansprüche, mit Zeittoreinrichtungen zur Isolierung der gewünschten Signale von Rauschsignalen.

**Revendications**

1. Système de formation d'image tomographique par onde ultrasonore comportant un transducteur d'onde ultrasonore (23) qui émet des signaux d'ondes ultrasonores dans un objet et qui reçoit des signaux d'ondes ultrasonores réfléchis par l'objet, un ensemble de lentilles ultrasonores (22a, 22d) pour focaliser les signaux émis sur un plan tomographique voulu ($X_1$, $X_1'$) de l'objet et pour focaliser des signaux réfléchis par le plan sur le transducteur (23), un dispositif à prismes tournants (22b, 22c) qui, en tournant, entraîne que le plan tomographique ($X_1$, $X_1'$) soit balayé par les signaux émis et focalisés, et un dispositif de compensation (23b; 70, 75) destiné à compenser la déviation du faisceau focalisé reçu au transducteur (23) résultant de la rotation du dispositif à prismes (22b, 22c) dans le temps entre le passage des signaux d'ondes émis et réfléchis à travers celui-ci, caractérisé en ce que le dispositif de compensation change la région effective de réception sur le transducteur en fonction de la rotation des prismes.

2. Système de formation d'image tomographique par onde ultrasonore selon la revendication 1, dans lequel ledit dispositif de compensation est formé par une plaque barrière à fente (70) entre le transducteur (23) et ledit ensemble de lentilles (22a, 22d), et ladite plaque (70) étant agencée pour être décalée de façon appropriée par rapport à la rotation du dispositif à prismes (22b, 22c) de manière à permettre que les signaux reçus voulus passent par la fente (75) vers le transducteur (23).

3. Système de formation d'image tomographique par onde ultrasonore selon la revendication 1, dans lequel le transducteur (23) est constitué par un réseau en trame d'éléments transducteurs (23, 23a, 23b) et dans lequel le dispositif de compensation assure que seules passent les sorties des éléments transducteurs dans la région effective sur laquelle les signaux voulus sont focalisés.

4. Système de formation d'image tomographique par onde ultrasonore selon l'une quelconque des revendications précédentes, comprenant un dispositif à porte de temporisation destiné à isoler les signaux voulus des signaux parasites.

# Fig. I

# Fig. 2

(A)

(B)

(C)

# Fig. 3

(A)

(B)

# Fig. 4